Europäisches Patentamt

**European Patent Office**

Office européen des brevets

(11) Publication number: **0 079 054**
A1

(12) **EUROPEAN PATENT APPLICATION**

(21) Application number: **82110168.0**

(22) Date of filing: **04.11.82**

(51) Int. Cl.³: **A 61 K 31/70**
**A 61 K 45/06**

(30) Priority: **10.11.81 JP 179953/81**

(43) Date of publication of application:
**18.05.83 Bulletin 83/20**

(84) Designated Contracting States:
**CH DE FR GB LI NL**

(71) Applicant: **Yamasa Shoyu Kabushiki Kaisha**
**10-1, Araoi-Cho 2-Chome**
**Choshi-Shi Chiba-Ken(JP)**

(72) Inventor: **Nakatsugawa, Shigekazu**
**1355 Willard Street**
**San Francisco California 94143(US)**

(74) Representative: **Dr. Elisabeth Jung Dr. Jürgen**
**Schirdewahn Dr. Gerhard Schmitt-Nilson Dr. Gerhard B.**
**Hagen Dipl.-Ing. Peter Hirsch**
**Clemensstrasse 30 Postfach 40 14 68**
**D-8000 München 40(DE)**

(54) **Enhancer of antitumor effect.**

㉟ An antitumor effect enhancer for antitumor agents comprising 9-β-D-arabinofuranosyl adenine, a chemotherapeutic comprising a combination of said enhancer with an antitumor agent and a use thereof are disclosed.

EP 0 079 054 A1

ᴵᴺSABETH JUNG
RGEN SCHIRDEWAHN
RHARD SCHM.TT-I.ᴵᴸᴸSON
ERHARD B. HAGEN +
-ING. PETER HIRSCH
ATENTANWÄLTE
ısstr. 30, 8000 München 40
Telefon: (089) 34 50 67

1

ℓ 0ℓ C

ENHANCER OF ANTITUMOR EFFECT

## BACKGROUND OF THE INVENTION

### Field of the art

This invention relates to a pharmaceutical for enhancing the antitumor effect of chemotherapeutics.

### Prior art

In general, in the field of chemotherapy of tumors, multiple anti-tumor agents have been combined to be used for the following purposes and effects:

1) By using in combination a number of different agents selected from those of alkylating agents, antimetabolites, antibiotics and alkaroids, which show mutually no cross resistancy and are different in mechanism of action, the anti-tumor effect can be enhanced additively or synergistically against tumors which are composed of a mixture of tumor cells different in sensitivity to various agents.

2) By using in combination anti-tumor agents different in the way to attack tumor cells which are proliferating at random, various stages in the cell cycle of tumor cells can be widely attacked to ensure complete killing of tumor cells.

3) By using not only agents different in mechanism of action but also those having relatively similar mechanisms of action, a synergistic effect can be expected. For example, by using in combination a number of agents which are blocking a series of steps participating in DNA synthesis, a strong synergistic effect can be exhibited.

4) Each anti-tumor agent has its specific side effect. Thus, by using in combination a number of agents with different side effects each in a dosage less than the limit above which side effects appear, the anti-tumor effect can be expected to be increased additively or synergistically while the side effects are dispersed.

By such a multi-agent combination treatment, it has been made possible to obtain an effect which could not be produced by using a single anti-tumor agent. However, each of the agents used in combination in such an application is an anti-tumor agent which can be independently used.

There have also been various attempts to use in combination with an anti-tumor agent a compound which does not per se have an anti-tumor effect for the purpose of strengthening the effect of the anti-tumor agent by preventing the anti-tumor agent from being inactivated in bodies. For example, it is known to use cytidine or uridine in combination with 1-β-D-arabinofuranosylcytosine (hereinafter referred to as "ara-C"), as disclosed in Japanese Laid-Open Patent Application No. 24150/1980. It is also known to use tetrahydrouridine, which is an inhibitor against cytidinedeaminase, in combination with ara-C, as disclosed in Cancer Research Vol. 30, p.2166 - 2172, 1970. Further, there is known another method wherein 5-fluorouracil (hereinafter referred to as "5-FU") or a derivative thereof is combined with a pyrimidine compound such as, for example, uracil, cytosine, thymine, orotic acid, 5-bromouracil, 5-iodouracil, 1-acetyluracil, 1-(2-tetrahydrofuryl)-uracil, 3-benzoyluracil, 1-cyclohexycarbamoyluracil, 1-n-hexycarbamoyluracil, uridine, 2'-deoxyuridine, 5-bromo-2'-deoxyuridine, cytidine, or 2'-deoxycytidine.

On the other hand, 9-β-D-arabinofuranosyl adenine (hereinafter abbreviated as "ara-A") has proved to exhibit cytostatic activity against cancer cells as a result of inhibiting DNA polymerase α, primarily through conversion to ara-ATP in living bodies, or to exhibit antiviral activity by inhibiting, for example, HSV-DNA polymerase. However, ara-A is readily deaminated by adenosine deaminase existing widely in living bodies to be converted to less active 9-β-D-arabinofuranosyl hypoxanthine, and their activities are not so remarkable. Particularly, with respect to antitumor activity, no remarkable cytostatic action against cancer cells can be recognized unless under the co-presence with a potent adenosine deaminase inhibitor such as 2'-deoxycoformycin or coformycin, even in experiments on the cell level. Further, according to in vivo experimentation, ara-A alone can exhibit only very weak activity against representative experimental tumors having potent adenosine deaminase activity. In fact, no attempt to apply clinically ara-A alone as an antitumor agent has been successful, and no attempt has been practiced to use ara-A in combination with other antitumor agents.

### SUMMARY OF THE INVENTION

I have made extensive studies on pharmaceuticals to enhance the antitumor effect of a chemotherapeutic in therapy of malignant tumors with a chemotherapeutic and have consequently found that ara-A has a marked effect of enhancing the antitumor effect of an antitumor agent. The present invention is based on this finding. The present invention thus concerns an enhancer of antitumor effect comprising ara-A as an active ingredient, which is to be used at the place for the treatment of malignant tumors with antitumor agents for enhancement of the antitumor effect thereof.

In another aspect of the present invention, there is provided a method of enhancement of antitumor effect which comprises administering ara-A to a tumor-bearing animal under the antitumor treatment with an antitumor agent.

According to the present invention, the antitumor effect of an antitumor agent is enhanced by ara-A. Since ara-A itself has almost no antitumor activity as mentioned above, such an activity of enhancing antitumor effect of ara-A has been entirely unexpected. Further, the chemotherapeutic comprising a combination of ara-A and an antitumor agent can be said to advantageously exhibit the synergistic effect of both agents.

### BRIEF DESCRIPTION OF THE DRAWING

In the accompanying drawing, the single figure is a graph indicating the results of tumor sizes (diameter ratios) measured with elapse of time in Test Example 3 of the present invention, wherein line 1 indicates the group treated with a combination of ACNU and ara-A, line 2 the group treated with

ACNU alone, line 3 the group treated with ara-A alone, and line 4 the control group (no treatment with pharmaceutical).

DETAILED DESCRIPTION OF THE INVENTION

Examples of antitumor agents whose antitumor effects can be enhanced by the pharmaceutical of the present invention are as follows.

(1) Antimetabolites

Examples of antimetabolites include methotrexate, 6-mercaptopurine, 5-FU and its derivatives [5-fluorouridine, 5-fluoro-2'-deoxyuridine, 1-$\beta$-D-arabinofuranosyl-5-fluorocytosine, 1-(2-tetrahydrofuryl)-5-fluorouracil (hereinafter referred to as "FT-207"), 1-(n-hexylcarbamoyl)-5-fluorouracil, 1-ethoxymethyl-5-fluorouracil, 1-ethoxycarbonyl-5-fluorouracil, 5-fluoro-5'-deoxyuridine, etc.], and ara-C and its derivatives (cyclocytidine, $N^4$-palmitoyl ara-C, $N^4$-stearoyl ara-C, $N^4$-behenoyl ara-C, ara-C-5'-stearyl phosphate, ara-C-oleyl phosphate, etc.).

(2) Antitumor-antibiotics

Examples of antitumor-autibiotics includes bleomycin (hereinafter referred to as "BLM"), neocalcinostatin, anthracycline type antibiotics (daunomycin, adriamycin, aclacynomycin, etc.).

(3) Alkylating agents

Examples of alkylating agents include nitrosourea such as ACNU, BCNU, CCNU, MCCNU, etc., 3'-[3-(2-chloroethyl)-3-nitrosoureido]-3'-deoxythymidine, and 3'-(3-methyl-3-nitrosoureido)-3'-deoxythymidine. ACNU stands for 1-(4-amino-2-methylpyrimidin-5-yl)methyl-3-(2-chloroethyl)-3-nitrosourea hydrochloride; BCNU for 1,3-bis(2-chloroethyl)-1-nitrosourea; CCNU for 1-(2-chloroethyl)-3-cyclohexyl-1-nitrosourea; and MCCNU for 1-(2-chloroethyl)-3-(4-methylcyclohexyl)-1-nitrosourea.

It is not required to set specific conditions for the method of administration and the dosage of the antitumor agent when used in combination with the pharmaceutical of the present invention, but optimum conditions for respective antitumor agents may conveniently be employed. The pharmaceutical of the present invention can also be used in the treatment of malignant tumors wherein administration of antitumor agents and radiotherapy are used in combination.

The pharmaceutical of the present invention may be administered to a patient under the antitumor treatment with an antitumor agent, namely simultaneously with, before or after administration of an antitumor agent.

The pharmaceutical of the present invention may be administered according to conventional systemic or topical administration. As the unit dosage form for administration of the pharmaceuticals, various forms depending on the purpose of treatment and the method of administration may be chosen. For example, as the forms to be employed for the systemic administration method, preparations can be formed in tablets, capsules, granules or solutions for oral administration and in injections or suppositories for parenteral administration, while they can be formed in slow-release capsules, ointments, injections or others for topical application. Such dosage unit forms can be prepared with the use of a carrier conventionally used in the field of pharmacology according to the preparation methods well known in the art. It is also possible to use various elaborations suitable for the object of the present invention. Further, as an embodiment of the invention, the antitumor agent to be used in combination and ara-A can be prepared as a formulation within the same dosage unit form. In this case, the pro portions of respective components to be formulated are determined according to the respective kinds employed.

The pharmaceutical of the present invention is employed in a quantity effective for enhancement of the antitumor activity. More specifically, as determined from the basic effectiveness experiments, the daily dose of the present pharmaceutical, in general, is preferably 20 to 3,000 mg/day for oral administration, 0.5 to 500 mg/day for injection, and 20 to 2,000 mg for suppository. The optimum effective amount should be determined according to judgement by the attendant physician, depending on the type of the antitumor agent, its dosage, the condition of the disease and the afflicted portion.

It is also possible to use the pharmaceutical of the present invention in combination with other enhancers of antitumor effect, either in an integrated dosage form or separate dosage forms.

It may be considered that ara-A enhances the anti-tumor effect by inhibiting repair of the damage of the tumor cells caused after exhibition of the pharmocological activity of an antitumor agent.

The pharmacological effect of the pharmaceutical according to the present invention will now be demonstrated by way of test data. The pharmocological test method is as described below.

C3H/He-strain male mice (8 weeks old) were inoculated intradermally in the right thigh with $4 \times 10^5$ cells of syngeneic squamous cell carcinoma SQ1. On the 13th or 14th day after inoculation of the tumor cells and thereafter, physiological saline solutions (or suspensions) of respective pharmaceuticals were administered intraperitoneally in respective dosages consecutively for respective days. After initiation of the treatment with the pharmaceuticals, the tumor sizes in the mice tested were measured in the three coordinate axial directions every day or every other day, and the results with respect to the following items were compared with those of the Control group.

$$(1) \ \text{Average diameter of tumor} = \left( \frac{\substack{\text{Max.} \\ \text{longitudinal} \\ \text{diameter}} + \substack{\text{Max.} \\ \text{lateral} \\ \text{diameter}} + \substack{\text{Max.} \\ \text{height} \\ \text{diameter}}}{3} \right)$$

$$(2) \ \text{Diameter ratio} = \frac{\substack{\text{Average diameter of tumor} \\ \text{at the time of measurement}}}{\substack{\text{Average diameter of tumor} \\ \text{at initiation of treatment} \\ \text{with pharmaceuticals}}}$$

$$(3) \ \text{Volume ratio} = (\text{Diameter ratio})^3$$

Test Example 1

As the antitumor agent, BLM was employed. Administrations of pharmaceuticals were performed at 15 mg/kg for BLM and/or 80 mg/kg for ara-A, consecutively for three days, and ara-A was administered 20 minutes after administration of BLM. The results obtained 8 days after initiation of the treatment with the pharmaceuticals are shown in Table 1.

Table 1

| Pharmaceutical treatment | | Average tumor diameter at initiation of the treatment (mm) | 8 days after initiation of the treatment with pharmaceuticals | | |
|---|---|---|---|---|---|
| Antitumor agent | Pharmaceutical of the invention | | Average diameter (mm) | Diameter ratio | Volume ratio |
| Control | – (n=8) | 6.50 ± 1.46 | 11.23 ± 1.46 | 1.78 ± 0.37 | 6.38 ± 4.78 |
| BLM | – (n=8) | 6.06 ± 1.48 | 9.50 ± 2.21 | 1.57 ± 0.13 | 3.96 ± 1.06 |
| – | ara-A (n=8) | 6.64 ± 1.44 | 11.49 ± 2.65 | 1.74 ± 0.18 | 5.38 ± 1.81 |
| BLM | ara-A (n=7) | 6.23 ± 1.82 | 8.43 ± 3.53 | 1.31 ± 0.32 | 2.35 ± 1.26 |

Test Example 2

As the antitumor agent, FT-207 was employed. Administrations of pharmaceuticals were performed at 100 mg/kg for FT-207 and/or 33 mg/kg for ara-A, consecutively for 11 days, and ara-A was administered one hour after administration of FT-207. The results obtained 14 days after initiation of the treatment with the pharmaceuticals are shown in Table 2.

Table 2

| Pharmaceutical treatment | | Average tumor diameter at initiation of the treatment (mm) | 14 days after initiation of the treatment with pharmaceuticals | | |
|---|---|---|---|---|---|
| Antitumor agent | Pharmaceutical of the invention | | Average diameter (mm) | Diameter ratio | Volume ratio |
| Control | – (n=8) | 6.20 ± 0.98 | 13.85 ± 2.67 | 2.31 ± 0.59 | 13.66 ± 9.24 |
| FT-207 | – (n=10) | 6.34 ± 0.57 | 8.63 ± 1.25 | 1.36 ± 0.17 | 3.04 ± 1.86 |
| – | ara-A (n=15) | 6.63 ± 0.78 | 17.29 ± 1.99 | 2.61 ± 0.30 | 17.22 ± 5.71 |
| FT-207 | ara-A (n=10) | 6.92 ± 0.57 | 6.96 ± 1.84 | 0.99 ± 0.21 | 1.12 ± 0.71 |

Test Example 3

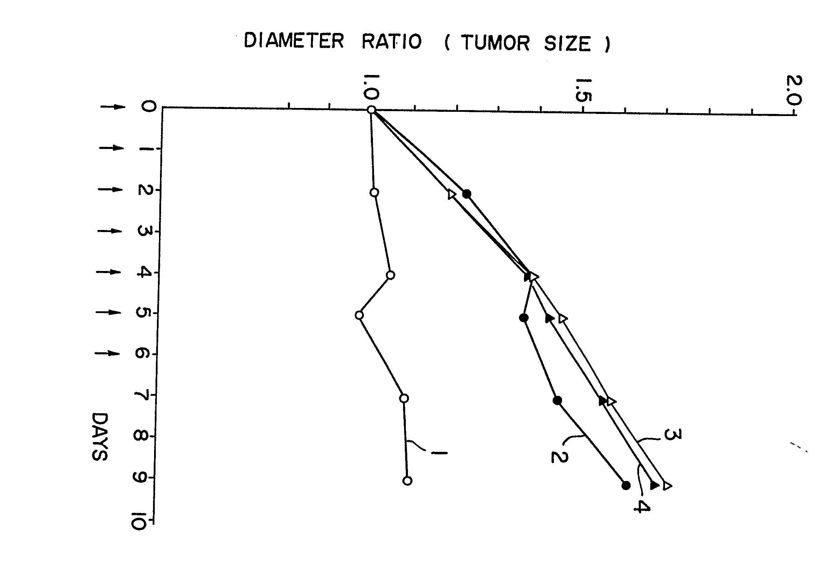

As the antitumor agent, ACNU was employed. Administrations of pharmaceuticals were performed at 12 mg/kg for ACNU and/or 33 mg/kg for ara-A, consecutively for 7 days, and ara-A was administered one hour after administration of ACNU. The results obtained 9 days after initiation of the treatment with the pharmaceuticals are shown in Table 3. The results of measurement of the tumor sizes with elapse of time from initiation of the treatment with the pharmaceuticals to the 9th day are also shown in FIG. 1.

## Table 3

| Pharmaceutical treatment | | Average tumor diameter at initiation of the treatment (mm) | 9 days after initiation of the treatment with pharmaceuticals | | |
|---|---|---|---|---|---|
| Antitumor agent | Pharmaceutical of the invention | | Average diameter (mm) | Diameter ratio | Volume ratio |
| Control | — (n=5) | 7.48 ± 1.17 | 12.64 ± 2.14 | 1.69 ± 0.06 | 4.82 ± 0.51 |
| ACNU | — (n=6) | 6.52 ± 1.41 | 10.53 ± 2.56 | 1.62 ± 0.19 | 4.41 ± 1.82 |
| — | ara-A (n=5) | 7.64 ± 0.60 | 13.08 ± 1.34 | 1.72 ± 0.19 | 5.21 ± 1.55 |
| ACNU | ara-A (n=6) | 7.22 ± 1.56 | 7.87 ± 1.77 | 1.10 ± 0.19 | 1.42 ± 0.73 |

12

0079054

13

CLAIMS:

1.      An antitumor effect enhancer for antitumor agents, comprising 9-β-D-arabinofuranosyl adenine as active ingredient.

2.      The antitumor effect enhancer according to Claim 1, wherein the antitumor agents are selected from the group consisting of antimetabolites, antitumor-antibiotics and alkylating agents.

3.      A preparation for use as an antitumor effect enhancer for antitumor agents which comprises 9-β-D-arabinofuranosyl·adenine and a pharmaceutically acceptable carrier.

4.      A chemotherapeutic, comprising a combination of 9-β-D-arabinofuranosyl adenine and an antitumor agent.

5.      A chemotherapeutic according to Claim 4, wherein the antitumor agents are selected from the group consisting of antimetabolites, antitumor-antibiotics and alkylating agents.

6.      A method of enhancement of antitumor effect which comprises administering to a tumor-bearing animal under the antitumor treatment with an antitumor agent 9-β-D-arabinofuranosyl adenine as an antitumor effect enhancer.

7.      In a method of treatment of one or more tumors in a tumor-bearing animal, the improvement which comprises administering to the tumor-bearing animal under an antitumor treatment with an antitumor agent 9-β-D-arabinofuranosyl adenine as an antitumor effect enhancer.

0079054

0079054

**European Patent Office**

**PARTIAL EUROPEAN SEARCH REPORT**
which under Rule 45 of the European Patent Convention
shall be considered, for the purposes of subsequent
proceedings, as the European search report

Application number

| DOCUMENTS CONSIDERED TO BE RELEVANT | | | EP 82 11 0168 |
|---|---|---|---|
| Category | Citation of document with indication, where appropriate, of relevant passages | Relevant to claim | CLASSIFICATION OF THE APPLICATION (Int. Cl. ³) |
| X | MARTIN NEGWER: "Organisch-Che-mische Arzneimittel und ihre Synonyma", edition 5, vol.I, 1978 Akademie-Verlag, BERLIN (DE)<br><br>* page 243, nr. 1347 "9-β-D-Ara-binofuranosyladenin" * | 1-3 | A 61 K 31/70<br>45/06 |
| X | FR - M - 3 585M (SOCIETE INDUS-TRIELLE POUR LA FABRICATION DES ANTIBIOTIQUES)<br><br>* page 2, right-hand column - page 3, abstract * | 1-3 | |
| X | CHEMICAL ABSTRACTS, vol.74, no.13, March 29, 1971, page 207, abstract no.63035p<br>./. | | **TECHNICAL FIELDS SEARCHED (Int. Cl. ³)**<br><br>A 61 K<br>C 07 H |

**INCOMPLETE SEARCH**

The Search Division considers that the present European patent application does not comply with the provisions of the European Patent Convention to such an extent that it is not possible to carry out a meaningful search into the state of the art on the basis of some of the claims.

Claims searched completely: 1-5
Claims searched incompletely:
Claims not searched: 6-7
Reason for the limitation of the search:

Method for treatment of the human or animal body by surgery or therapy (see article 52(4) of the European Patent Convention).

| Place of search | Date of completion of the search | Examiner |
|---|---|---|
| The Hague | 10-02-1983 | BRINKMANN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03 82

European Patent Office

**PARTIAL EUROPEAN SEARCH REPORT**

| | DOCUMENTS CONSIDERED TO BE RELEVANT | | CLASSIFICATION OF THE APPLICATION (Int Cl ) |
|---|---|---|---|
| Category | Citation of document with indication, where appropriate, of relevant passages | Relevant to claim | |
| | COLUMBUS OHIO (US) <br> & Progr. Antimicrob. Anticancer Chemother., Proc. Int. Congr. Chemother.,6th 1969 (Pub.1970), 2, 180-4 <br> GARILHE, MICHEL et al.: "Effect of some arabinosides and their associations on Ehrlich ascites tumor development in mice" <br><br> * the whole document * | 4-5 | |
| A | CHEMICAL ABSTRACTS, vol.92, no.1, January 7, 1980, page 24, abstract no.256n <br> COLUMBUS OHIO (US) <br> & Cancer Res. 1979, 39(9), 3655-60 <br> W. PLUNKETT et al.: "Biochemical basis of the increased activity of 9-$\beta$-D-arabinofuranosyladenine in the presence of inhibitors of adenosine deaminase." | 1-5 | TECHNICAL FIELDS SEARCHED (Int. Cl.³) |
| A | CHEMICAL ABSTRACTS, vol.87, no.3, July 18, 1977, page 16, abstract no.15742p <br> COLUMBUS OHIO (US) <br> & Ann. N.Y. Acad. Sci.1977, 284, 91-102 <br> W. PLUNKETT et al.: "Increased toxicity of 9-$\beta$-D-arabinofuranosyladenine in the presence of an inhibitor of adenosine deaminase." | 1-5 | |
| A | US - A - 4 148 889 (VIDYA S. GUPTA) <br><br> * column 2, lines 49-67 * | 1-5 | |